# EUROPÄISCHE PATENTANMELDUNG

(11) **EP 3 395 254 A1**
(43) Veröffentlichungstag der Anmeldung: **31.10.2018**
(21) Anmeldenummer: 18165637.2
(22) Anmeldetag: 04.04.2018
(51) Int. Cl.: A61B 6/00

(54) **HALTERUNG FÜR EINEN DETEKTORTRÄGER AN EINEM RÖNTGENGERÄT**

(30) Priorität: 27.04.2017 AT 9317 U
(71) Anmelder: Kapun, Christian, 9061 Klagenfurt am Wörthersee (AT)
(72) Erfinder: Kapun, Christian, 9061 Klagenfurt am Wörthersee (AT)
(74) Vertreter: Beer & Partner Patentanwälte KG

(57) **Zusammenfassung**

Eine Halterung (1) für einen Träger (2) für einen Detektor (3) und/oder für einen Röntgenraster eines Röntgengerätes (4) ist dazu vorgesehen, den Träger (2) in vorgegebener Relativlage zum Röntgengerät (4) zu halten. Um den Träger (2) abnehmen zu können, ist die Halterung (1) am Röntgengerät (4) über eine Kupplung (7) lösbar befestigt. Die Kupplung (7) befindet sich zwischen einem Arm (8), der von einem an dem Tragarm (5) des Röntgengerätes (4) montierten Aufnahmeteil (6) absteht, und dem oberen Ende (15) eines Bügels (14) der Halterung (1), der an seinem in Gebrauchslage unteren Ende (16) den Träger (2) für einen Detektor (3) und/oder für einen Röntgenraster trägt.

## Beschreibung

Die Erfindung betrifft eine Halterung für einen Träger für einen Detektor und/oder einen Röntgenraster eines Röntgengerätes, wobei die Halterung an dem Strahlertragarm des Röntgengerätes angeordnet ist, und wobei der Träger in vorgegebener Relativlage zum Röntgengerät gehalten ist.

Das Behandlungsergebnis bei mehrfach verletzten, polytraumatisierten Patienten wird ganz wesentlich von einer reibungslosen Abfolge der Arbeitsabläufe im Bereich Primärversorgung beeinflusst. Dies erfordert neben dem Stabilisieren der Vitalparameter des Patienten eine rasche Diagnose und Therapie vitalbedrohender Verletzungen.

Bei mehrfach verletzten Patienten, insbesondere bei Wirbelverletzungen, kann jedes Umlagern des Patienten die ursprüngliche Verletzung verschlimmern.

Aus diesem Grund ist es notwendig, Röntgenuntersuchungen durchführen zu können, bei denen der Patient nicht angehoben werden muss, um den Detektor des Röntgengerätes unter den Patienten schieben zu können.

Bekannt sind Halterungen von Trägern für Detektoren und einen gegebenenfalls erforderlichen Röntgenraster, die einen U-förmigen Bügel aufweisen und den Träger in einem fixen, zentrierten Abstand von der Röntgenröhre halten. So kann der Detektor mit dem Träger an dem U-Bügel unter einen röntgenstrahltransparenten Tisch, auf dem der zu untersuchende Patient liegt, bewegt werden, ohne dass der Patient selbst bewegt werden muss.

Aus WO 2014/022840 A2 ist ein C-förmiger Träger für einen Detektor einer Röntgenapparatur bekannt, an dem auch das Röntgengerät selbst angeordnet ist. Der Detektor wird von seiner Halterung, die am C-förmigen Träger angeordnet ist, abgenommen, wenn er unter einem Patienten in Stellung gebracht werden soll.

Eine ähnliche Anordnung eines Detektors eines Röntgengerätes ist aus WO 2014/055488 A2 bekannt.

DE 26 51 830 A1 zeigt ein Röntgengerät, bei dem über einem Röntgentisch eine Bildverstärkeranordnung angeordnet ist. Die Bildverstärkeranordnung ist mit dem Röntgentisch verstellbar über eine Teleskop-Verbindungsarmanordnung verbunden.

Problematisch ist bei bekannten Halterungen, dass diese an dem Röntgengerät fix montiert sind, so dass die Halterung bei den normalen Röntgenuntersuchungen störend im Weg ist.

Der Erfindung liegt die Aufgabe zugrunde, eine Halterung der eingangs genannten Gattung zur Verfügung zu stellen, die zusammen mit dem Träger für den Detektor und/oder den Röntgenraster eines Röntgengerätes problemlos abgenommen werden kann.

Gelöst wird diese Aufgabe erfindungsgemäß mit einer Halterung, welche die Merkmale von Anspruch 1 aufweist.

Bevorzugte und vorteilhafte Ausgestaltungen der Erfindung sind Gegenstand der Unteransprüche.

Da die erfindungsgemäße Halterung an dem Röntgengerät, insbesondere dessen Strahlertragarm, über eine lösbare Befestigung gehalten ist, kann sie bei Bedarf einfach abgenommen werden. Das Röntgengerät kann dann ohne die Halterung und den an ihr angeordneten Träger für einen Detektor oder einen Röntgenraster verwendet werden.

Besonders vorteilhaft ist es im Rahmen der Erfindung, wenn an dem Röntgengerät, insbesondere dessen Strahlertragarm, ein Aufnahmeteil vorgesehen ist, an dem die Halterung über eine Kupplung befestigt werden kann.

Besonders bevorzugt ist es im Rahmen der Erfindung, wenn die Kupplung eine selbsttätig verriegelnde Kupplung ist, so dass das Anbringen der Halterung am Röntgengerät einfach und ohne komplizierte Handhabungen möglich ist.

Weitere Einzelheiten und Merkmale der Erfindung ergeben sich aus der nachstehendenden Beschreibung eines bevorzugten Ausführungsbeispiels anhand der Zeichnungen. Es zeigt:

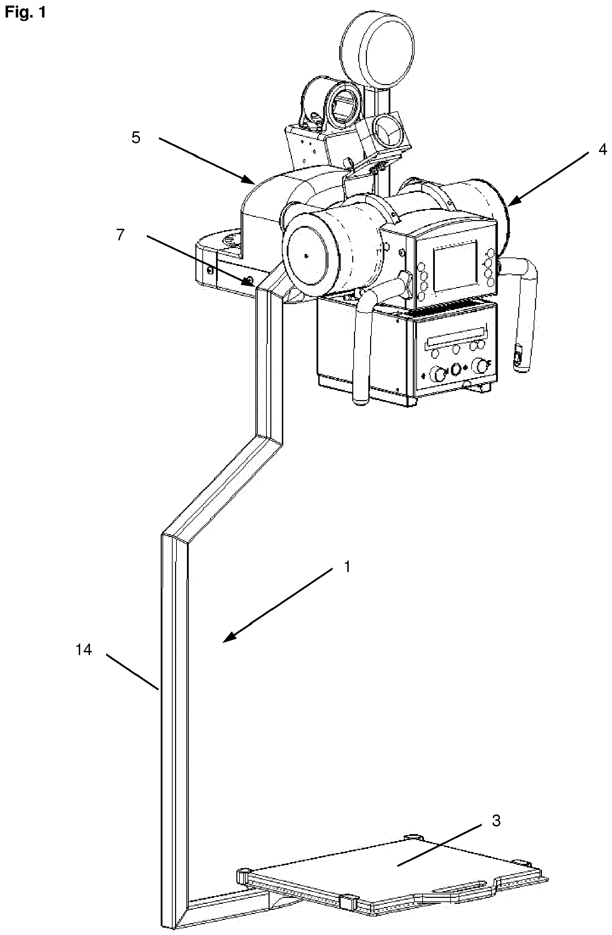

- Fig. 1: eine Halterung, die an einem Röntgengerät angebracht ist und

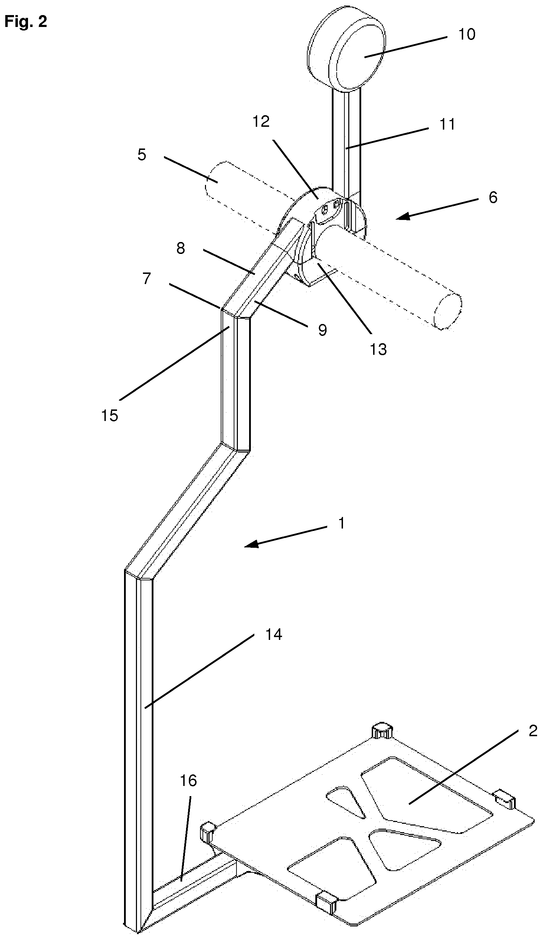

- Fig. 2: die Halterung ohne das Röntgengerät.

Eine Halterung 1 umfasst einen mehrfach gewinkelten Bügel 14, der mit seinem oberen Ende 15 über eine Kupplung 7 an einem Tragarm 5 eines Röntgengerätes 4 befestigt werden kann. Die Halterung 1 trägt an ihrem unteren Ende 16 einen Träger 2, auf dem ein Detektor 3 und wahlweise auch ein Röntgenraster eingesetzt oder eingeschoben werden kann.

Die erfindungsgemäße Halterung 1 umfasst einen Aufnahmeteil 6, der im Wesentlichen ringförmig ausgebildet ist und einen oberen halbringförmigen Teil 12 und einen unteren halbringförmigen Teil 13 aufweist. Der Aufnahmeteil 6 umschließt den Tragarm 5 des Röntgengerätes 4.

An dem oberen halbringförmigen Teil 12 des Aufnahmeteils 6 ist über einen Arm 11 ein Gegengewicht 10 angeordnet.

Der Aufnahmeteil 6 umfasst weiters einen Arm 8. Der Arm 8 geht vom oberen halbringförmigen Teil 12 aus. An dem freiem Ende 9 des Armes 8 ist ein Teil der Kupplung 7 angeordnet. Ein zweiter Teil der Kupplung 7 ist an dem in Gebrauchslage oberen Ende 15 des Bügels 14 der Halterung 1 angeordnet.

Die Kupplung 7 ist so ausgebildet, dass sie beim Zusammenführen der Enden 9 und 15 selbsttätig verriegelt, also das obere Ende 15 des Bügels 14 mit dem Arm 8 verbindet.

Beispielsweise ist vorgesehen, dass im Bereich des freien Endes 9 oder im Bereich des freien Endes 15 ein Körper (nicht gezeigt) vorgesehen ist, der in dem gegenüberliegenden Teil des Armes 8 bzw. des oberen Endes 15 einführbar ist. Zusätzlich kann die Kupplung 7 einen federbelasteten oder federnden Teil aufweisen, der mit einem Verriegelungszapfen in den Körper eingreift, so dass die Kupplung 7 geschlossen (verriegelt) ist. Durch elastisches Verformen oder Wegbiegen des Verriegelungsteils aus der Verrastungsstellung kann die Kupplung 7 wieder geöffnet werden, wenn der Bügel 14 der Halterung 1 vom Aufnahmeteil 6 abgenommen werden soll.

Der Kupplung 7 kann ein elektrischer Kontakt zugeordnet sein. Der Kontakt verbindet einen im Bügel 14, also dem abnehmbaren Teil der erfindungsgemäßen Halterung 1, vorgesehenen Schalter, der als Druckschalter ausgebildet sein kann, mit einer dem Stativ des Röntgengerätes 4 zugeordneten, elektrischen Verriegelung, die, wenn sie aktiviert ist, das Verschieben des Röntgengerätes 4 verhindert. Mit Hilfe dieses Schalters kann die Verriegelung des Röntgengerätes 4 entriegelt werden und das Röntgengerät 4 kann frei bewegt (verschoben) werden.

Ein Vorteil der erfindungsgemäßen Halterung 1 ist es, dass sie an Röntgenanlagen beliebiger Hersteller angebracht werden kann, wobei es allenfalls nur erforderlich ist, den Aufnahmeteil 6 entsprechend den Abmessungen und der äußeren Form des Tragarmes 5 des Röntgengerätes 4 angepasst auszubilden.

Zusammenfassend kann ein Ausführungsbeispiel der Erfindung wie folgt beschrieben werden:
Eine Halterung 1 für einen Träger 2 für einen Detektor 3 und/oder für einen Röntgenraster eines Röntgengerätes 4 ist dazu vorgesehen, den Träger 2 in vorgegebener Relativlage zum Röntgengerät 4 zu halten. Um den Träger 2 abnehmen zu können, ist die Halterung 1 am Röntgengerät 4 über eine Kupplung 7 lösbar befestigt. Die Kupplung 7 befindet sich zwischen einem Arm 8, der von einem an dem Tragarm 5 des Röntgengerätes 4 montierten Aufnahmeteil 6 absteht, und dem oberen Ende 15 eines Bügels 14 der Halterung 1, der an seinem in Gebrauchslage unteren Ende 16 den Träger 2 für einen Detektor 3 und/oder für einen Röntgenraster trägt.

## Patentansprüche

1. Halterung (1) für einen Träger (2) für einen Detektor (3) und/oder für einen Röntgenraster eines Röntgengerätes (4), wobei die Halterung (1) an dem Strahlertragarm (5) des Röntgengerätes (4) angeordnet ist, und wobei der Träger (2) in vorgegebener Relativlage zum Röntgengerät (4) gehalten ist, **dadurch gekennzeichnet, dass** die Befestigung der Halterung (1) am Röntgengerät (4), insbesondere dessen Strahlertragarm (5), lösbar ist.

2. Halterung nach Anspruch 1, **dadurch gekennzeichnet, dass** am Röntgengerät (4), insbesondere dessen Strahlertragarm (5), ein Aufnahmeteil (6) vorgesehen ist, an dem die Halterung (1) lösbar befestigt ist.

3. Halterung nach Anspruch 2, **dadurch gekennzeichnet, dass** eine Kupplung (7) vorgesehen ist, die beim Anbringen der Halterung (1) am Aufnahmeteil (6) verriegelbar ist, insbesondere selbsttätig verriegelt, um die Halterung (1) an dem Röntgengerät (4), insbesondere dessen Strahlertragarm (5), festzulegen.

4. Halterung nach Anspruch 3, **dadurch gekennzeichnet, dass** ein Teil der Kupplung (7) an dem Ende (9) eines Armes (8) des Aufnahmeteils (6) und der andere Teil der Kupplung (7) an dem am Röntgengerät (4), insbesondere dessen Strahlertragarm (5), festzulegenden Ende (15) der Halterung (1) vorgesehen ist.

5. Halterung nach einem der Ansprüche 2 bis 4, **dadurch gekennzeichnet, dass** an dem Aufnahmeteil (6) ein Gegengewicht (10) vorgesehen ist.

6. Halterung nach Anspruch 5, **dadurch gekennzeichnet, dass** das Gegengewicht (10) mit Abstand von dem Aufnahmeteil (6) angeordnet ist.

7. Halterung nach Anspruch 6, **dadurch gekennzeichnet, dass** das Gegengewicht (10) an dem Aufnahmeteil (6) über einen Arm (11) gehalten ist.

8. Halterung nach einem der Ansprüche 2 bis 7, **dadurch gekennzeichnet, dass** der Aufnahmeteil (6) ringförmig und den Strahlertragarm (5) umgreifend ausgebildet ist.

9. Halterung nach Anspruch 8, **dadurch gekennzeichnet, dass** der Aufnahmeteil (6) zwei im Wesentlichen halbringförmige Teile (12, 13) umfasst.

10. Halterung nach Anspruch 9, **dadurch gekennzeichnet, dass** das Gegengewicht (10), insbesondere der das Gegengewicht (10) haltende Arm (11), und/oder der Arm (8) des Aufnahmeteils (6) von dem in Gebrauchslage oberen Teil (12) des Aufnahmeteils (6) abstehend angeordnet ist.

11. Halterung nach einem der Ansprüche 1 bis 10, **dadurch gekennzeichnet, dass** die Halterung (1) einen im Wesentlichen U-förmigen, mehrfach abgewinkelten oder abgebogenen Bügel (14) aufweist, und dass der Träger (2) an dem in Gebrauchslage vom Röntgengerät (4) entfernt liegenden Ende (16) des Bügels (14) angeordnet ist.

12. Halterung nach einem der Ansprüche 3 bis 11, **dadurch gekennzeichnet, dass** die Kupplung (7) durch wenigstens einen federnden oder federbelasteten Verriegelungsteil verriegelbar ist.

13. Halterung nach Anspruch 12, **dadurch gekennzeichnet, dass** die Kupplung (7) einen in den am Aufnahmeteil (6) vorgesehenen Arm (8) oder in das Ende (15) des Bügels (14) der Halterung (1) einführbaren Körper aufweist.

14. Halterung nach Anspruch 13, **dadurch gekennzeichnet, dass** der Verriegelungsteil bei verriegelter Kupplung (7) in den Körper eingreift.
